Europäisches Patentamt

European Patent Office

Office européen des brevets

(11) Veröffentlichungsnummer : **0 116 725 B1**

# EUROPÄISCHE PATENTSCHRIFT

(45) Veröffentlichungstag der Patentschrift : 18.06.86

(51) Int. Cl.[4] : **C 07 C 46/04**, C 07 C 50/16

(21) Anmeldenummer : 83201590.3

(22) Anmeldetag : 08.11.83

(54) **Verfahren zur Herstellung von Phenanthrenchinon.**

(30) Priorität : 18.02.83 DE 3305528

(43) Veröffentlichungstag der Anmeldung : 29.08.84 Patentblatt 84/35

(45) Bekanntmachung des Hinweises auf die Patenterteilung : 18.06.86 Patentblatt 86/25

(84) Benannte Vertragsstaaten :
CH DE FR GB IT LI

(56) Entgegenhaltungen :
DE-B- 1 240 065
ORGANIC SYNTHESE, Band 34, 1954, Seiten 76-78, John Wiley & Sons, Inc., New York, US; R. WENDLAND u.a.: "Phenanthrenequinone"
CHEMICAL ABSTRACTS, Band 51, 1957, Spalte 16392-e, Columbus, Ohio, US; K. SHIMPACHIRO: "Synthesis of phenanthrenequinone by the oxidation of phenanthrene with chromic acid"

(73) Patentinhaber : **Rütgerswerke Aktiengesellschaft
Mainzer Landstrasse 217
D-6000 Frankfurt a.Main 1 (DE)**

(72) Erfinder : **Tonnius, Dietrich
Pfungstadter Strasse 8
D-6800 Mannheim 31 (DE)**
Erfinder : **Weiss, Wolfgang, Dr.
Kelterweg 3
D-6803 Neckarhausen (DE)**
Erfinder : **Orth, Winfried, Dr.
Am Schachtelgraben 28
D-6735 Hassloch/Pfalz (DE)**
Erfinder : **Miele, Heinrich
Roonstrasse 1
D-6940 Weinheim (DE)**
Erfinder : **Pastorek, Emmerich, Dr.
Grünbergerstrasse 90
D-6944 Hemsbach (DE)**

**0 116 725**

## Beschreibung

Die Erfindung betrifft ein Verfahren zur Herstellung von 9,10-Phenanthrenchinon durch Oxidation von Phenanthren mit Chromsäure in schwefelsaurem Medium.

Diese Reaktion ist aus Organic Synthesis, Vol. 34, S. 76, und aus DE-PS 1 240 065 bekannt.

Bei dem Verfahren gemäß Organic Synthesis heizt sich das Reaktionsgemisch durch Reaktionswärme auf und die Umsetzung erfolgt schließlich unter Rückfluß beim Siedepunkt des Wassers. Die Ausbeuten an Phenanthrenchinon betragen 44 bis 48 %.

Demgegenüber wird nach DE-PS 1 240 065 die Oxidation bei Temperaturen zwischen 100 und 200 °C unter Druck durchgeführt. Durch diese Verfahrensführung wird eine wesentliche Steigerung der Phenanthrenchinonausbeute bis auf 85 % der Theorie erhalten. Das resultierende Phenanthrenchinon enthält aber noch 12 bis 14 % nichtumgesetztes Phenanthren, das in einem zusätzlichen Reinigungsprozeß abgetrennt werden muß. Abgesehen davon, daß das Verfahren unter Druck durchgeführt wird, hat es somit den Nachteil, daß die Oxidationsreaktion nicht vollständig abläuft.

Es ist daher Aufgabe der vorliegenden Erfindung, ein Verfahren zur Herstellung von Phenanthrenchinon zu entwickeln, das diese Nachteile nicht zeigt, das ohne Verwendung von Druck eine vollständige Oxidation des Phenanthrens ermöglicht und das somit eine höhere Ausbeute an Phenanthrenchinon liefert.

Die Lösung der Aufgabe erfolgt durch ein Verfahren gemäß der Ansprüche 1-3.

Überraschenderweise wurde gefunden, daß die Oxidation des Phenanthrens leicht und vollständig erfolgt, wenn man aus Wasser, Alkalibichromat und Phenanthren bei 95-100° eine Dispersion herstellt, diese Dispersion auf 80-85 °C abkühlt, danach durch Zugabe von konzentrierter Schwefelsäure die Reaktion in Gang bringt und die Reaktionstemperatur im Bereich von 80-85 °C hält.

Es wurde weiterhin gefunden, daß die Menge an benötigtem Chromat und Schwefelsäure geringer ist, als dies bisher üblich war, obwohl die Oxidationsreaktion weitgehender verläuft.

Weitere überraschende Vorteile des erfindungsgemäßen Verfahrens liegen darin, daß die Qualität des Einsatzproduktes nahezu unbedeutend ist. Das Verfahren läßt sich auch mit Phenanthren minderer Qualität durchführen, ohne daß Fehlchargen entstehen, die aufgrund von allzu großen Harzbildungen nicht mehr aufgearbeitet werden können. Außerdem fällt beim erfindungsgemäßen Verfahren das Phenanthrenchinon nicht mehr in Form von Kügelchen oder Klumpen an, die organische und anorganische Verunreinigungen einschließen, sondern vielmehr als feines, kristallines Produkt, das sich leicht filtrieren und waschen läßt.

Die praktische Durchführung des Verfahrens besteht darin, daß eine Mischung aus beispielsweise 1 Mol Phenanthren mit 1,1 bis 2 Mol Natriumbichromat und 300-700 ml Wasser auf 95-100 °C erhitzt und dabei durch intensives Mischen dispergiert wird.

Zur Stabilisierung der Dispersion können dem Gemisch geringe Mengen eines Netzmittels zugegeben werden. Der Einsatz eines Netzmittels erlaubt es, mit niedrigeren Rührgeschwindigkeiten zu dispergieren und auch während der folgenden Reaktion nur mäßig zu rühren. Arbeitet man ohne Netzmittel, so muß das Reaktionsgemisch während der gesamten Reaktion durch mechanisches Dispergieren entweder mittels hochtouriger Rühr- oder Schwinggeräte oder mit Hilfe von Beschallung mit hohen Frequenzen dispers gehalten werden, so daß ein Absetzen einzelner Bestandteile verhindert wird.

Als Netzmittel können alle handelsüblichen nichtionischen, anionischen oder kationischen Hilfsmittel dienen, wie z. B. Fettsäureester, Fettamine, Fettsäureamide, Polyamine, Polyglykoläther, Carboxylate, fluorierte Carboxylate, Naphthenate, Sulfonate, Sulfate, Phosphate oder quartäre Ammoniumverbindungen. Diese Netzmittel werden dem dispergierten oder zu dispergierenden Gemisch in einer Menge von 0,05-0,5 g pro Mol Phenanthren zugegeben.

Die bei 95-100 °C hergestellte Dispersion wird auf 80-85 °C abgekühlt. Erst danach wird die Oxidationsreaktion durch Zugabe von konzentrierter Schwefelsäure gestartet und das Reaktionsgemisch wird durch kontrollierte, dosierte Säurezugabe und durch äußere Kühlung auf einer Temperatur von 80-85 °C gehalten. Dieser Verfahrensschritt dauert im allgemeinen 2 bis 3 Stunden. Danach folgt eine etwa einstündige Nachreaktion des stark sauren Reaktionsgemisches bei 80-85 °C. Anschließend wird abgekühlt auf 50-70 °C und das ausgefallene Phenanthrenchinon in an sich bekannter Weise abfiltriert, gewaschen und getrocknet.

### Beispiele

### Beispiel 1

178 g Phenanthren (86 %ig) werden mit 500 g $Na_2Cr_2O_7 \cdot 2H_2O$ und 400 g Wasser vermischt.

Die Mischung wird mit 0,1 g Tetraäthylammoniumperfluoroctansulfonat versetzt und unter Rühren (180 U/min) auf 100 °C aufgeheizt und danach auf 80 °C abgekühlt.

Unter weiterem Rühren werden innerhalb von 2 Stunden 950 g konzentrierte Schwefelsäure zugegeben und die Temperatur des Reaktionsgemisches durch äußere Kühlung im Temperaturbereich von 80-85 °C gehalten.

2

Danach wird 1 Stunde bei 85 °C nachgerührt und anschließend das Gemisch auf 50 °C abgekühlt. Das ausgefallene 9,10-Phenanthrenchinon wird von der schwefelsauren Mutterlauge abgetrennt und mit Wasser gewaschen, bis das Waschwasser nahezu neutral abläuft.

Nach dem Trocknen werden 175,5 g feinkristallines Phenanthrenchinon erhalten mit einer Reinheit von 92 % und einer Ausbeute von 90 % der Theorie, bezogen auf eingesetztes Phenanthren. Das Produkt ist frei von nicht umgesetztem Phenanthren.

Beispiele 2-6

Analog Beispiel 1 wird Phenanthren zu Phenanthrenchinon oxidiert, wobei jeweils einzelne Bedingungen verändert werden.

Die folgende Aufstellung zeigt diese Änderungen und die bei den einzelnen Beispielen erhaltenen Ausbeuten an Phenanthrenchinon und die Reinheiten.

| Beispiel | Änderung | Ausbeute/Gehalt |
|---|---|---|
| 2 | Reaktion bei $100^{\circ}$C (ohne Abkühlen auf $80^{\circ}$C) | 86 % / 80 %ig |
| 3 | analog Beispiel 2, kein Netzmittel | 85 % / 80 %ig |
| 4 | 0,2 g Diisobutylnaphthalinsulfonat als Netzmittel | 89 % / 92 %ig |
| 5 | Einsatz von 82%igem Phenanthren | 88 % / 89 %ig |
| 6 | Herstellung und Aufrechterhaltung der Dispersion mit Ultraschall, kein Netzmittel | 90 % / 92 %ig |

## Patentansprüche

1. Verfahren zur Herstellung von 9,10-Phenanthrenchinon durch Oxidation von Phenanthren mittels Chromsäure durch Zutropfen von konzentrierter Schwefelsäure zu einer Mischung von in Wasser suspendiertem Phenanthren und Alkalibichromat, dadurch gekennzeichnet, daß bei 95-100 °C eine Dispersion aus Phenanthren, Alkalibichromat und Wasser hergestellt wird, diese Dispersion auf 80 bis 85 °C abgekühlt und die Oxidationsreaktion unter Kühlung und kontrollierter Zugabe von konzentrierter Schwefelsäure im Temperaturbereich von 80 bis 85 °C durchgeführt wird.

2. Verfahren nach Anspruch 1, dadurch gekennzeichnet, daß der Dispersion aus Phenanthren, Alkalibichromat und Wasser 0,05-0,5 g eines Netzmittels pro Mol Phenanthren zugegeben werden.

3. Verfahren nach Anspruch 1, dadurch gekennzeichnet, daß die Dispersion aus Phenanthren, Alkalichromat und Wasser durch mechanisches Dispergieren hergestellt und während der Reaktion aufrechterhalten wird.

## Claims

1. Process for the production of 9,10-phenanthrenequinone by oxidizing phenanthrene with the aid of chromic acid by controlled addition of concentrated sulfuric acid to a mixture of phenanthrene suspended in water and alkali bichromate, comprising the steps of preparing a dispersion of phenanthrene, alkali bichromate and water at 95-100 °C, cooling said dispersion down to 80 to 85 °C and carrying out the oxidation reaction under cooling and controlled addition of concentrated sulfuric acid in a temperature range from 80 to 85 °C.

2. The process of claim 1, further comprising adding 0.05-0.5 g of a surface active agent per mole phenanthrene to the dispersion of phenanthrene, alkali bichromate and water.

3. The process of claim 1, further comprising forming said dispersion of phenanthrene, alkali bichromate and water by mechanical dispersion and initiating the mechanical dispersion during the reaction.

**0 116 725**

**Revendications**

1. Procédé pour la préparation de 9,10-phénanthrène-quinone par oxydation de phénanthrène au moyen d'acide chromique par addition goutte à goutte d'acide sulfurique concentré à un mélange de phénanthrène et de bichromate alcalin en suspension dans l'eau, caractérisé en ce que à 95-100 °C est produite une dispersion de phénanthrène, de bichromate alcalin et d'eau, cette dispersion est refroidie à 80 jusqu'à 85 °C et la réaction d'oxydation est effectuée sous refroidissement et addition contrôlée d'acide sulfurique concentré dans la plage de températures de 80 à 85 °C.

2. Procédé selon la revendication 1, caractérisé en ce que l'on ajoute à la dispersion de phénanthrène, de bichromate alcalin et d'eau, 0,05-0,5 g d'un agent mouillant par mole de phénanthrène.

3. Procédé selon la revendication 1, caractérisé en ce que la dispersion de phénanthrène, de bichromate alcalin et d'eau est préparée par dispersion mécanique et maintenue pendant la réaction.

4